# EUROPÄISCHE PATENTSCHRIFT

(11) **EP 0 815 811 B1**
(45) Veröffentlichungstag und Bekanntmachung des Hinweises auf die Patenterteilung: **26.05.2004**
(21) Anmeldenummer: 97110923.6
(22) Anmeldetag: 02.07.1997
(51) Int. Cl.: A61F 2/52

(54) **Epithese**
Epithesis
Epithèse

(30) Priorität: 05.07.1996 DE 19627112
(43) Veröffentlichungstag der Anmeldung: 07.01.1998
(73) Patentinhaber: augmentec Aktiengesellschaft Implantologie-Epithetik, 91799 Langenaltheim (DE)
(72) Erfinder: Gehl, Gerolf, Dr. med. dent., 8700 Küsnacht (CH)
(74) Vertreter: Thiel, Christian, Dr. Dipl.-Chem.

(56) Entgegenhaltungen:
- EP-A- 0 686 380
- GB-A- 2 121 291
- GB-A- 2 262 041
- US-A- 4 401 492
- US-A- 4 701 230

## Beschreibung

Die Erfindung betrifft eine Epithese, die aus einer Hülle, die rückseitig an ein Körperteil angepaßt ist und frontseitig die erwünschte Körperoberfläche nachbildet, sowie einer die Hülle ausfüllenden Füllung besteht.

Epithesen zum Ausgleich von angeborenen oder erworbenen Körperdefekten sind seit langem bekannt und vielfach beschrieben. Sie werden vielfach eingesetzt, um die Folgen von Unfällen oder chirurgischen Eingriffen zu verdecken und eine natürliche Körperoberfläche vorzuspiegeln. Ein häufiger Einsatzzweck ist die Nachbildung der weiblichen Brust nach der chirurgischen Entfernung, bedingt durch bösartige Tumoren.

Epithesen dienen dabei zum einen der Abdeckung der defekten Körperoberfläche gegen unerwünschte äußere Einflüsse, also auch beispielsweise zur Unterstützung der Wundheilung und zur Verbesserung der Hygiene. Zum anderen werden Epithesen aber vor allem aus kosmetischen Gründen verschrieben und getragen.

In Anbetracht des Verwendungszwecks ist es erforderlich, die Epithese optimal an die Körperoberfläche anzupassen. Dies deshalb, weil nur auf diese Art und Weise die medizinischen und hygienischen Rahmenbedingungen erfüllt werden können und weil nur auf diese Weise der erforderliche "Tragekomfort" gewährleistet werden kann. Hinzu kommt, daß nur eine gut sitzende Epithese auch den erwünschten optischen Eindruck erweckt und es dem Patienten erlaubt, sich damit ungezwungen zu bewegen. Eine optimale Nachbildung der gewünschten Körperoberfläche ist dabei selbstverständlich, einschließlich der Nachbildung der natürlichen Hautfarbe.

Herkömmliche Epithesen bestehen aus einer äußeren Hülle, die an die Körperoberfläche angepaßt ist und mit einer Flüssigkeit gefüllt ist. Das Hüllmaterial besteht zumeist aus Silikonkautschuk oder auch einem Polyurethankunststoff, die sich als außergewöhnlich hautfreundlich erwiesen haben. Die Füllung besteht häufig aus einem flüssigen Silikon. Die flüssige Füllung gewährleistet dabei eine gewisse Verformbarkeit über die ebenfalls aus einem elastischen Material bestehende Hülle.

Nachteilig hat sich bei diesen bekannten Epithesen aber das relativ hohe Gewicht erwiesen. Dieses relativ hohe Gewicht erfordert besondere Maßnahmen bei der Befestigung der Epithese, die dadurch aufwendiger gestaltet sein muß, als normalerweise als angenehm empfunden wird. Dies, wie auch das hohe Gewicht, führen zu Beeinträchtigungen bei der Beweglichkeit und, bei stärkerer Bewegung, auch zum Verrutschen der Epithese. Ferner besteht die Gefahr des Auslaufens bei Verletzung der Hülle. Luftgefüllte Epithesen fallen bei Verletzung der Hülle in sich zusammen. Eine Reparatur und dauerhafte Wiederherstellung ist in beiden Fällen nicht einfach.

EP-A-0 686 380 beschreibt eine Epithese des eingangs beschriebenen Art, bei der ein Teil der Füllung aus einem elaztischen Material einer Dichte von d < 0,25 g/cm³ besteht. Hierbei kann Silikonschaumstoff zusammen mit Silikongel verwandt werden.

Der Erfindung liegt nun die Aufgabe zugrunde, ein Material für die Epithesenfüllung bereitzustellen, das die oben geschilderten Nachteile vermeidet, dennoch in medizinischer Hinsicht unbedenklich ist, hautfreundlich ist - für den Fall des Durchtritts durch die Hülle oder der Verletzung der Hülle - und der Epithese die Elastizität der natürlichen Hautoberfläche zu verleihen vermag. Des weiteren soll die Epithese über lange Zeiträume formstabil bleiben.

Diese Aufgabe wird mit einer Füllung gelöst, die aus einem eingeschäumten Silikonschaumstofft einer Dichte d < 0,25 g/cm³ besteht.

Wesentlich ist, daß die Füllung elastisch eingestellt ist, d. h. auf Druck bis zu einem gewissen Grade in sich nachgeben kann, ohne daß dies zum Verrutschen der Epithese führt. Aufgrund der Verwendung von Flüssigkeiten in bekannten Epithesen ist diese Elastizität nicht gewährleistet, da Flüssigkeiten praktisch nicht komprimierbar sind und immer, unter Weitergabe des Drucks, seitlich auszuweichen trachten. Die erfindungsgemäß zum Einsatz kommenden Materialien sind dagegen so elastisch, daß der ausgeübte Druck zumindest teilweise vom Füllungsmaterial aufgefangen werden kann.

Besonders geeignet ist eine Füllung, die aus einem einzigen integrierten Schaumstoffteil besteht. Eine solche Prallschaumfüllung kann zwar nich seitlich verlagert werden, ist aber in sich nachgiebig. Erfingungsgemäß besteht dieses Schaumstoffteil aus einem integralen Silikonschaumstoff, d. h. das Schaumstoffteil wird innerhalb der Hülle durch Einbringen eines Schaumbildners erzeugt, sodaß eine vollständige Ausschäumung gewährleistet ist.

Als Materialien hierfür kommen an und für sich bekannte Zwei-Komponenten-Silikonschaumbildner in Frage, die aus einer Silikonkomponente und einem Katalysator bestehen. Durch Vermischung der beiden Komponenten entsteht innerhalb kurzer Zeit der gewünschte Schaum. Werden die beiden Komponenten unmittelbar nach dem Vermischen in die Hülle eingebracht, entsteht der Schaum in der Hülle selbst und füllt diese vollständig und "prall" aus. Ein geeignetes Material ist beispielsweise der "Prosthetic Foam A-2380" (silicon foam elastomere base) der Fa. Silicon in Lakeside, AZ, USA. Der entsprechende Katalysator wird unter der Bezeichnung A-2380 katalysts von der gleichen Firma vertrieben.

Das Hüllmaterial besteht vorzugsweise aus einem hautfreundlichen, medizinisch getesteten Silikonkautschuk, der einfärbbar ist und kommerziell erhältlich ist. Typischerweise hat der Kautschuk eine Härte Shore 20 A. Ein brauchbares Material wird beispielsweise von der Fa. Orthomax, United Kingdom, vertrieben.

Da die erfindungsgemäßen Epithesen eine poröse Füllung aufweisen, weisen sie ein ausgesprochen geringes Gewicht auf. Eine Steuerung des Gewichts ist ohne weiteres möglich, indem Einfluß auf den Porositätsgrad genommen wird. Da keine flüssige Füllung vorhanden ist, ist ein Auslaufen nicht möglich, wie es beispielsweise als Folge von langjährigem Gebrauch oder durch mechanische Beanspruchung bei herkömmlichen Epithesen möglich ist; ebensowenig kann ein Zusammenfallen eintreten, wie bei den bekannten luftgefüllten "Schwimmprothesen".

Die erfindungsgemäßen Epithesen werden unter Verwendung der folgenden Schritte hergestellt:
- Fertigung einer Hülle anhand eines Körperabdrucks, wobei die Hülle mit den zum Befüllen notwendigen Öffnungen ausgestattet ist;
- Einbringen einer Füllung aus einem 2K-Silikonschaumbildner und Ausschäumen zu einer Dichte von d < 0,25 g/cm³;
- Verschließen der wenigstens einen Öffnung mit einem Stopfen.

Erfindungsgemäß ist vorgesehen, ein integrales eingeschäumtes Silikonschaumstoffteil im Inneren der Hülle zu erzeugen. Dies erfolgt dadurch, daß die miteinander reagierende Masse aus Schaumbildner und Katalysator bzw. reaktiver zweiter Komponente in das Innere der Hülle eingespritzt und dort abreagieren gelassen wird. Es entsteht auf diese Art und Weise ein die Hülle prall ausfüllendes Schaumstoffteil, das der Epithese die erwünschte Elastizität verleiht. Dabei kann durch Verwendung geeigneter Katalysatoren auch eine reaktive Anbindung des im Inneren ausgebildeten Silikonschaumstoffs an die Silikonkautschukhülle erreicht werden, was die Epithese sehr straff erscheinen läßt, was beispielsweise bei Verwendung im Bereich der Waden sinnvoll sein kann. Je nach Art der Einbringung des Schaumbildners kann es erforderlich sein, die Epithesenhülle mit mehrerern Öffnungen zum Druckausgleich zu versehen. Zum Ausschäumen mit dem Schaumbildner ist das Küvettieren zweckmäßig, d. h. das Einbetten der hülle in eine Form, um den Schäumungsdruck aufzufangen und die äußere Form zu stabilisieren.

Nach dem Einschäumen der Hülle werden die in der Hülle vorhandenen Öffnungen mit geeigneten Stopfen aus beispielsweise Silikonkautschuk verschlossen, wobei es sinnvoll ist, eine Verklebung der Stopfen mit dem Hüllenmaterial zu erreichen, um eine Einsiegelung des Inhalts zu gewährleisten.

Die erfindungsgemäßen Epithesen werden in erster Linie für die Korrektur des Erscheinungsbildes der weiblichen Brust nach Operationen eingesetzt. Sie können aber auch an anderen Körperteilen Verwendung finden, beispielsweise im Bereich des Gesäßes, der Taille oder der Waden. Brust-Epithesen werden zweckmäßigerweise in den BH, das Mieder oder den Badeanzug eingearbeitet, wodurch der richtige Sitz gewährleistet ist.

## Patentansprüche

1. Epithese, bestehend aus einer Hülle, die rückseitig an ein Körperteil angepaßt ist und frontseitig die erwünschte Körperoberfläche nachbildet, und einer die Hülle ausfüllenden Füllung, **dadurch gekennzeichnet, daß** die Füllung aus einem eingeschäumten Silikonschaumstoffteil einer Dichte von d < 0,25 g/cm³ besteht.

2. Epithese nach Anspruch 1, **dadurch gekennzeichnet, daß** die Hülle aus einem hautverträglichen Silikonkautschuk oder Polyurethankunststoff besteht.

3. Brustepithese nach einen der vorstehenden Ansprüche.

4. Verfahren zur Herstellung der Epithese nach einem der vorstehenden Ansprüche, welches die folgenden Schritte umfaßt:
- Fertigung einer Hülle anhand eines Körperabdrucks, wobei die Hülle mit den zum Befüllen notwendigen Öffnungen ausgestattet ist;
- Einbringen einer Füllung aus einem 2K-Silikonschaumbildner und Ausschäumen zu einer Dichte von d < 0,25 g/cm³;
- Verschließen der wenigstens einen Öffnung mit einem Stopfen.

5. Verfahren nach Anspruch 4, **dadurch gekennzeichnet, daß** die Hülle mit einem Stopfen aus Silikonkunststoff verschlossen wird, der mit dem umgebenden Hüllenmaterial verklebt wird.

## Claims

1. An epithesis consisting of a sheath adapted to a body part on the back and simulating the desired body surface on the front, and a filling inside the sheath, wherein the filling consists of a foamed-in silicone foam part with a density of d < 0.25 g/cm³.

2. The epithesis of claim 1 wherein the sheath consists of skin-compatible silicone rubber or polyurethane plastic.

3. A breast epithesis according to any of the above claims.

4. A method for producing the epithesis of one of the above claims which includes the following steps:
producing a sheath using a body impression, the sheath being equipped with the openings necessary for filling;
introducing a filling consisting of a two-component silicone foam-forming agent and foaming to a density of d < 0.25 g/cm³;
sealing the at least one opening with a stopper.

5. The method of claim 4, wherein the sheath is sealed with a stopper made of silicone plastic which is bonded with the surrounding sheath material.

## Revendications

1. Epithèse composée d'une douille qui est adaptée au dos à une partie du corps et qui reproduit à l'avant la surface souhaitée du corps, et d'un remplissage de la douille, **caractérisée en ce que** le remplissage est composé d'une partie expansée en mousse de silicone d'une densité de d < 0,25 g/cm³.

2. Epithèse selon la revendication 1, **caractérisée en ce que** la douille est composée d'un caoutchouc au silicone compatible avec la peau ou d'une matière plastique au polyuréthanne.

3. Epithèse de la poitrine selon l'une des revendications précédentes.

4. Procédé de fabrication de l'épithèse selon l'une des revendications précédentes, qui comprend les étapes suivantes :
- fabrication d'une douille à l'aide d'une empreinte du corps, la douille étant munie des ouvertures nécessaires au remplissage,
- introduction d'un remplissage en formateur de mousse au silicone 2K et expansion à une densité de d < 0,25 g/cm³,
- fermeture de l'ouverture au nombre d'au moins une avec un bouchon.

5. Procédé selon la revendication 4, **caractérisé en ce que** la douille est fermée avec un bouchon en matière plastique au silicone, qui est collé avec le matériau voisin de la douille.
